# EUROPEAN PATENT SPECIFICATION

(11) **EP 3 348 578 B1**
(45) Date of publication and mention of the grant of the patent: **09.10.2019**
(21) Application number: 15906481.5
(22) Date of filing: 22.10.2015
(51) Int. Cl.: C07K 19/00, C07K 14/525, C12N 15/28, C12N 15/70, A61K 38/17, A61P 35/00, C07K 14/705, A61K 38/00

(54) **DOUBLE-TARGET MUTEIN MUR6S4TR OF TRAIL, AND PREPARATION METHOD AND USE THEREOF**
DOPPEL-TARGET-MUTEIN MUR6S4TR VON TRAIL SOWIE HERSTELLUNGSVERFAHREN UND VERWENDUNG DAVON
MUTÉINE MUR6S4TR DE TRAIL À DOUBLE CIBLE, SON PROCÉDÉ DE PRÉPARATION ET SON UTILISATION

(43) Date of publication of application: 18.07.2018
(73) Proprietor: Chengdu Huachuang Biotechnology Co., Ltd, Chengdu, Sichuan 610041 (CN)
(72) Inventor: CHEN, Shouchun, Chengdu Sichuan 610041 (CN); XU, Qi, Chengdu Sichuan 610041 (CN); YAN, Juan, Chengdu Sichuan 610041 (CN); HUANG, Xianzhou, Chengdu Sichuan 610041 (CN); WEI, Lijia, Chengdu Sichuan 610041 (CN); HU, Haiyang, Chengdu Sichuan 610041 (CN)
(74) Representative: Sun, Yiming
(86) International application number: PCT/CN2015/092561
(87) International publication number: WO 2017/066963

(56) References cited:
- WO-A1-2011/161260
- WO-A2-2012/143477
- CN-A- 103 524 627
- CN-A- 104 628 863
- DATABASE EMBASE [Online] ELSEVIER SCIENCE PUBLISHERS, AMSTERDAM, NL; 12 December 2014 (2014-12-12), TAN J ET AL: "Cell-penetrating chimeric apoptotic peptide AVPI-LMWP/DNA co-delivery system for cancer therapy", XP002783589, Database accession no. EMB-2015826060 & TAN J ET AL: "Cell-penetrating chimeric apoptotic peptide AVPI-LMWP/DNA co-delivery system for cancer therapy", YAOXUE XUEBAO 20141212 ZHONGHUA YIXUEHUI ZAZHISHE CHN, vol. 49, no. 12, 12 December 2014 (2014-12-12), pages 1718-1723, ISSN: 0513-4870
- MAO, H.L. ET AL.: 'Smac Peptide Potentiates Trail- or Paclitaxel-Mediated Ovarian Cancer Cell Death in Vitro and in Vivo' ONCOLOGY REPORTS vol. 29, 28 February 2013, pages 515 - 525, XP055376315
- GUO, F. ET AL.: 'Ectopic Overexpression of Second Mitochondria-Derived Activator of Caspases (Smac/Diablo) or Cotreatment with N-Terminus of Smac/Diablo Peptide Potentiates Epothilone B Derivative-(BMS 247550) and Apo-2L/TRAIL-Induced Apoptosis' BLOOD vol. 99, no. 9, 05 January 2002, pages 3419 - 3426, XP055376316

## Description

### Technical Field

The invention relates to the field of genetic engineering drugs, and particularly to a bi-targeted mutein MuR6S4TR of TRAIL and a preparation method and application thereof. In the invention, the bi-targeted mutein MuR6S4TR of TRAIL has excellent therapeutic effects on multiple different types of tumor, and is a new generation of high-efficiency tumor cell apoptosis-inducing drugs with great potential.

### Background

### 1. Progress and significance of Apo 2L/TRAIL for oncotherapy

Tumor necrosis factor-related apoptosis-inducing ligand (TRAIL) is the member of Tumor necrosis factor (TNF) superfamily, and its gene sequences were independently cloned and obtained by Wiley et al. in 1995 and by Pitti et al. in 1996 respectively, and TRAIL was named as Apo 2 Ligand (Apo 2L) by Pitti et al. Later study confirmed that Apo 2L was essentially the same protein as TRAIL, so it is customarily called Apo 2L/TRAIL. TRAIL primarily functions as a congenital or acquired immunity modifier of organisms and secondly as an antitumor agent for immune surveillance in the cellular exogenous apoptotic pathway. The best advantage of TRAIL is that it can selectively introduce the apoptosis of various tumor cells and hardly has any toxicity to normal cells. Research data indicate that Apo 2L / TRAIL has an apoptosis-inducing effect on human tumor cell strains from various sources both in vitro and in vivo, including colon (rectal) cancer, lung cancer, breast cancer, prostatic cancer, pancreatic cancer, renal carcinoma, central nervous system tumor, thyroid cancer, lymphoma, leukemia and multiple myeloma.

TRAIL has been developed as an important potential antineoplastic drug for almost 20 years since it was discovered. Clinical trials of TRAIL have entered Phase II in foreign countries and Phase III has been completed in China. A lot of in vitro and in vivo tests confirm that TRAIL has tumor-specific cytotoxicity, and shows obvious synergism especially when it is used in combination with a low dose of chemotherapy drugs. On the contrary, the study shows that TRAIL resistance caused by the loss of apoptosis mechanism in organism is clearly related to the rapid growth and metastasis of tumor cells.

Tumors are a group of highly heterogeneous diseases. The traditional typing method based on tissues and organs and pathologic change is no longer appropriate for the diagnosis and treatment of tumors. The current research direction is to clarify the gene expression and molecular typing of different tumor cells, and treat the patient with targeted therapy. A deeper understanding of antineoplastic drugs enables people to understand that, the activation of tumor cell apoptosis pathway is involved in the process where cytotoxic drugs, molecular targeted drugs or monoclonal antibodies are in effect. The signal pathway that induces the tumor cell apoptosis is the pivotal and key link of these drugs, and apoptosis evasion is an important mechanism of tumor development and drug resistance.

### 2. Defects and countermeasures of Apo 2L/TRAIL for oncotherapy

Recent progress shows that only relying on Apo 2L/TRAIL for treating multiple different types of tumors is not enough. Although the recombination of agonistic monoclonal antibodies of Apo 2L/TRAIL or receptor DR4/DR5 of TRAIL have achieved encouraging results in clinical treatment of Phase I, but no clear clinical benefit has appeared in subsequent clinical study of phase II. A great number of studies show that normal cells and about half (even up to 60%) of passage tumor cell strains have drug resistance to TRAIL. According to the review by Roberta di Peitro and Giorgia zauli, Apo 2L/TRAIL is sensitive to 61 of 92 lines of studied primary or passage tumor cells, with sensitivity of 66.3%, while the remaining 31 lines are drug-resistant with drug resistance rate of 33.7%. The resistance of TRAIL to normal cells has its physiological significance. TRAIL is precisely regulated in vivo and only plays a role in removing aged and degenerated and transformed cells during growth and development, without killing normal cells. Almost all of the TRAIL-sensitive tumor cells have similar integrity and effects in all links and factors of apoptotic signaling pathway thereof, and each type of TRAIL-resistant tumor cells has some defects and mutations in some links and factors of the apoptotic signaling pathway. These drug-resistant tumor cell apoptotic thresholds abnormally rise due to these defects and mutations, therefore, these cells can easily avoid apoptosis so as to continuously grow and proliferate.

A large number of studies show that using only Apo 2L/TRAIL has no high-efficiency inhibition and killing effects for many tumor cells. The reason is that the tumor cell apoptotic signaling pathway is a very complex and large system, which contains many apoptosis-promoting factors and a large number of apoptosis inhibitors, and the interaction between such two factors determines the final destination of tumor cells. Soundness and effects of the apoptotic signaling pathway are necessary for tumor cell apoptosis, but not sufficient conditions. Multiple different types of drugs, molecules or gene interventions can enhance the sensitivity of TRAIL to tumor cells, these drugs comprise different types of chemotherapy drugs, natural products and small molecule kinase inhibitors. These drugs enhance the activity of TRAIL-induced tumor cell apoptosis by strengthening the cell exogenous apoptotic signaling pathway (e.g., up-regulating DRs expression, enhancing the aggregation and redistribution of DRs in lipid raft micro-area of cell membrane, enhancing the endocytosis of TRAIL/DRs compound in cell membrane, promoting the recruitment from DISC to TRAIL/DRs compound, activating the activity of Caspase 8 and inhibiting the activity of apoptotic antagonists FLIP, XIAP and lAPs) or by strengthening the mitochondrial apoptotic signaling pathway (e.g., enhancing the depolarization of mitochondrial potential, promoting the increase of mitochondria permeability and releasing Cyt c, Smac or ARTs, promoting splitting Bid into tBid and oligomerization of Bax and Bad, and inhibiting apoptotic antagonists Bcl-2, Bcl-xL, Bcl-w, Mcl-1 and surviving), or by inhibiting other cell survival signaling pathways (e.g., ERK/PI3K/AKt, MEK, Jak-STAT 3, MAPK and NF-κB) or combination of several pathways respectively.

Although TRAIL and its agonistic monoclonal antibodies have been temporarily frustrated in drug development, with the complete clarification of the apoptotic signaling pathway and the complete disclosure of the conversion relationship between apoptosis and resistance, the research and development of the targeting anticancer drugs based on apoptotic signaling pathway have not been stopped. At present, more studies are focusing on the combined application of TRAIL and cytotoxic drugs, but most of the experiments show that this combination can produce obvious synergistic effect on TRAIL-sensitive tumor cells only, but cannot completely reverse the drug-resistant phenomena caused by multiple different drug-resistant mechanisms. Since TRAIL and cytotoxic drugs belong to two different types of drugs, the drugs are different in drug dose, administration route and action mode, and it is less likely to develop a single, stable and controllable new drug. Moreover, TRAIL has toxic and side effect and unapparent advantage after being combined with cytotoxic drugs.

### 3. Design thought of Apo 2L/TRAIL bi-targeted mutein

The study shows that multiple proteins of Apoptosis inhibitor (lAPs) are potent inhibitors of tumor cell apoptosis. Overexpression of lAPs is found in many drug-resistant tumor cells, of which X-linked inhibitor of apoptosis (XIAP) is an important member of the lAPs and has a negative effect for regulating the apoptosis of various tumor cells.

Ducketl et al. cloned the XIAP gene encoding a protein containing 497 amino acids with a molecular weight of about 57 KD in 1996. XIAP, an important member of the apoptosis inhibitor, is a potent inhibitor of apoptosis signaling because its sequence contains multiple Baculovirus IAP repeats (BIRs). XIAP is expressed at high level in multiple drug-resistant tumor cells.

Du et al. identified and purified a protein in 2000, and named it as the Second mitochondrial-derived activator of caspase (Smac) which is also known in other literatures as Direct inhibitor of apoptosis-binding protein with low pi (DIABLO). The study by Chai et al. showed that Smac not only promoted the cleavage activation of Procaspase-3, but also enhanced the enzymatic activity of mature caspase-3. The process depended on the specific role of Smac and lAPs. The study on Smac crystal structure confirms that the Smac amino acid sequence at N-termin al is closely related to its effects, and 7 amino acids at N-terminal of Smac can be used alone in vitro to promote the activation of Procaspase-3. In order to further understand the molecular structure basis of the roles of Smac and lAPs, Liu et al. and Wu et al. determined the molecular structure of 9 amino acids with complete effects on XIAP and N-terminal of Smac respectively. The result showed that 4 amino acids (Ala-Val-Pro-Ile, AVPI) at N-terminal of Smac could identify the surface minor groove in BIR3 region of XIAP protein. The result suggests that the N-terminal sequence of Smac could represent that the whole protein with complete effects enables inhabitation of XIAP protein. Since 4 amino acids at the N-terminal of Smac and 4 amino acid sequences at N-terminal of Caspase-9 have same homology, Smac can competitively bind to the BIR3 region of XIAP and destroy the ligation of the Caspase-9 and the XIAP BIR3 fragment to release Caspase-9 and increase the activity of Caspase-9.

Mitochondrial-initiated downstream apoptotic events play an important role in the drug resistance of TRAIL-resistant tumor cells. Smac/DIABLO agonists enhance the sensitivity of TRAIL-resistant tumor cells to TRAIL primarily because Smac/DIABLO agonists can strongly inhibit the drug-resistance lAPs, especially the activity of XIAP, thereby enhancing the signal transduction of the mitochondria apoptosis pathway.

Various methods have been used to inhibit the activity of lAPs, in which Smac agonists increase the sensitivity of drug-resistant tumor cells to TRAIL-induced apoptosis by inhibiting the activity of the XIAP protein. Currently, the tumor types involved in the combination study of Smac agonists and TRAIL mainly comprise lung cancer, colon (rectal) cancer, breast cancer, liver cancer, pancreatic cancer, bladder cancer, prostate cancer, glioma, kidney cancer, melanoma, leukemia, nasopharynx cancer and ovarian cancer.

### Smac agonists include:

### (1) Smac cell-penetrating peptide fusion protein

Roa et al. observed that Smac-Tat peptide enhanced the sensitivity of drug-resistant glioma cells to TRAIL-induced apoptosis by inhibiting the activity of XIAP. Yang et al. observed that Smac N7 (R8) targeted the lAPs, especially XIAP to reverse the resistance of the drug-resistant lung cancer cells H460 to TRAIL.

### (2) Viral vector of Smac gene

Khorashadizadeh et al. transfected cells with hA-MSC-ST vector to secrete a new type of cell-penetrating Smac and trimer TRAIL that could obviously enhance the sensitivity of the drug-resistant breast cancer cells to TRAIL-induced apoptosis. Pei et al. used adenovirus vector to significantly reduce the XIAP level in liver cancer cells to completely remove the transplantation tumor cells of tumor-bearing animals. Wang et al. used the adenovirus vector ZD55-TRAIL-IETD-Smac containing TRAIL-IETD-Smac to completely clear the tumor cells from the hepatic transplantable tumor model animals.

### (3) Smac-4 or Smac-7 at N-terminal of Smac

Kandasamy et al. observed that previously treated Smac-7 at N-terminal of drug-resistant tube cells could reverse the resistance to drug-resistance tumor drugs treated cells and restore the sensitivity to TRAIL. Mao et al. observed that the combination of Smac N7 and TRAIL could significantly enhance the sensitivity of the transplantation tumor model of drug-resistant ovarian cancer cells A2780 to TRAIL, both of which had obvious synergistic effect. Yang et al. observed that Smac N7 (R8) targeted the lAPs, especially XIAP to reverse the resistance of the drug-resistant lung cancer cells H460 to TRAIL. Guo et al. obviously enhanced the PARP cleavage activity of TRAIL-induced caspase 3 and promoted tumor cell apoptosis by using Smac-7 or Smac-4 at N-terminal of Smac.

### (4) Small molecular simulant of Smac

Metwalli et al. found that Smac simulant had effect of inhibiting bladder cancer cell when being used alone, but could significantly enhance the anti-tumor effect of TRAIL when being combined with TRAIL. Wu et al. observed that the combination of Smac simulant and TRAIL could reduce the proportion of CSC-like cells in nasopharyngeal carcinoma cells SP and inhibit tumor stem cell cloning and formation of microsphere, and drug combination could remove CSC-like tumor cells from the animal transplantation tumor model. Allensworth et al. found that a bivalent Smac simulant Birinapant was used alone to induce the death of TRAIL-resistant SUM 190 (Erb2 overexpression) breast cancer cells, enhance TRAIL-sensitive SUM149 (triple-negative, EGFR activation) breast cancer cells on the sensitivity of TRAIL-induced apoptosis. Rockbrader et al. observed that Smac simulant significantly enhanced the inhibition effect of TRAIL on MDA-MB-231, T47D and MDA-MB-453 breast cancer cells. Huang et al. found that the combined application of TRAIL and Smac simulant could significantly enhance the ability to induce KRAS mutations in lung cancer apoptosis and had almost no effect on normal cells, and observed obvious reduction of tumor load in tumor-bearing animal from in vivo experiment. Lu et al. observed that Smac simulant SM-164 combined with TRAIL for sensitive cells or TRAIL non-sensitive cells of TRAIL, breast cancer, prostate cancer and colon (rectal) cancer could play a high synergistic effect, and SM-164 and TRAIL combined could rapidly remove transplantation tumor of animal from the in vivo model of the animal with breast cancer. In addition, Smac simulant can induce the death of drug-resistant ovarian cancer, liver carcinoma, leukemia and melanoma cells, and can overcome the increase of NF-κB activity caused by TRAIL and inhibit the activity of NF-κB simultaneously.

### (5) Smac siRNA

Vogler et al. knocked out the XIAP gene by RNA interference to induce pancreatic cancer cell apoptosis based on the synergism with TRAIL. In two in vivo transplantation tumor model experiments of animals with pancreatic cancer cells, inhibition of XIAP siRNA and combination with TRAIL could completely remove transplantation tumor. Chawla-Sarkar et al. observed that siRNAs interfered with XIAP or Survivin had strongest anti-tumor effect in melanoma cells, and observed the same result in renal cancer cells.

### (6) Smac-TRAIL fusion protein

Jerzy et al. ligated Smac-8 (AVPIAQKP) (see SEQ ID NO: 7), at N-terminal of Smac, R7 (RRRRRRR) (see SEQ ID NO: 8) cell-penetrating peptide structure, MMP2 and MMP9 and urokinase cleavage site (PLGLAGRVVR) sequence (see SEQ ID NO: 9), TRAIL (95-281aa) sequence encoded cDNA in order, and cloned them on prokaryotic expression vector pET30a to obtain recombinant expression vector pET30a-AD-O53.2. The expression vector was used to transform E. coli BL21 (DE3) with a good expression, and obtain the target protein by ion exchange purification. The median lethal dose of the fusion mutein 053.2 is fmol level for the most sensitive cells (including lung cancer, colon (rectal) cancer, pancreatic cancer, liver cancer, kidney cancer and urinary tract tumors, etc.), whereas non-transformed human umbilical vein endothelial cells (HUVEC), human or murine hepatocytes are not toxic. The fusion protein is well resistant to animals and can significantly inhibit the growth of colon (rectal) cancer and lung cancer cells in transplantation tumor animal models.

The combination of TRAIL-Smac bi-targeted therapy is used for treating different types of tumor, and can obviously enhance the sensitivity of different types of tumor cells or transplantation tumor models to drugs in vivo and in vitro, and enhance their antineoplastic activity, causing less toxic to normal cells.

Although the TRAIL and Sma combined treatment method has achieved a certain effect, but it is still far from the clinical application. In the development process of small molecule Smac agonist drugs, such method is not the optimal pharmaceutical mode due to the safety of small molecule drugs, differences with two different drugs of TRAIL, convenience of single use and stability of clinical efficacy. Due to the potential safety concerns of gene therapy, gene therapy based Smac agonistic strategy has remained to be clarified for a long time, and it still remains in the basic study phase so far, therefore, formation of a patent drug has little potential.

### Summary of the Invention

Document research shows that Smac N4 has the complete effect of Smac protein and is a potent inhibitor of a key TRAIL-resistant apoptosis inhibitor XIAP (X-linked inhibitor of apoptosis) that mainly presents in various drug-resistant tumor cells. Therefore, we hypothesize that the QRVA in the original TRAIL sequence is mutated into Smac-4 AVPI at N-terminal of Smac, and new constructed mutein is named as bi-targeted mutein MuR6S4TR of TRAIL closely following the RRRRRR (R6) sequence of TRAIL-MuR6 on the basis of cell-penetrating peptide-like mutant TRAIL-MuR6 of TRAIL using combinatorial biological means and methods. The bi-targeted mutein of TRAIL has synergistic effect with clear double targets for both the extracellular receptor apoptosis pathway of tumor apoptosis and the apoptosis inhibitor XIAP of multiple drug-resistant tumor cells. The effect mode of combination of multiple targets meets the up-to-date idea of the design for inducing tumor apoptosis drugs, and has better therapeutic effect on drug-resistant tumor with high expression of XIAP. In design of drug structure, we actively introduce the biomolecular marker of XIAP gene expression, preferably the tumor type with optimal molecular phenotype to hopefully improve the antineoplastic clinical efficacy.

The invention is to selectively mutate the ammonic acid sequences at site 8 to site 11, i.e., proline, glutamine, arginine, valine and alanine (QRVA) (see SEQ ID NO: 12) following RRRRRR (R6) (see SEQ ID NO: 10) at site 2 to site 7 in the MuR6 sequence into Smac-4 at N-terminal of Smac protein, i.e., alanine, valine, proline and isoleucine (AVPI) (see SEQ ID NO: 11) sequences, so as to make the new mutein have cell-penetrating peptide-like mutability and the activity of the Second mitochondrial-derived activator of caspase on the basis of cell-penetrating peptide-like mutant TRAIL-MuR6 of TRAIL (PCT/CN2015/073524: TRAIL cell-penetrating peptide-like mutant MuR6 of TRAIL, preparation method and application) sequence. The new mutein is named as bi-targeted mutein MuR6S4TR of TRAIL. The protein has synergistic effect with clear double targets for both the extracellular receptor apoptosis pathway of tumor apoptosis and the apoptosis inhibitor XIAP of multiple drug-resistant tumor cells.

Based on the defects of the prior art, the invention relates to a bi-targeted mutein MuR6S4TR of TRAIL, a preparation method and application thereof. The first purpose is to provide a novel bi-targeted mutein MuR6S4TR of TRAIL capable of greatly enhancing wild-type protein antineoplastic activity of TRAIL, specifically reversing the drug resistance of multiple drug-resistant tumor cells to the wild-type protein of TRAIL. The prepared mutein can not only rapidly take effect after directly entering cytoplasm by penetrating the cell membrane, but also promote the aggregation and internalization of the death receptor/mutein complex in the lipid raft micro-area of cell membrane, and enhance the transduction of exogenous apoptotic signaling pathway. The bi-targeted mutein MuR6S4TR of TRAIL has higher expression efficiency and better structural stability. The bi-targeted mutein MuR6S4TR of TRAIL has a superior therapeutic effect on various drug-resistant tumor cells, and is a new generation of high-efficiency tumor cell apoptosis-inducing drugs with great potential.

In order to achieve the above purpose, the technical solution of the invention is as follows: a bi-targeted mutein MuR6S4TR of TRAIL, wherein the amino acid sequence of the mutein is shown as SEQ ID NO: 2, enabling the site 2 to site 11 at N-terminal of mutein to become bi-targeted muteins containing cell-penetrating peptide sequence RRRRRR (R6) and binding sequence AVPI of apoptosis inhibitor XIAP by selectively mutating the ammonic acid sequences at site 8 to 11 site from QRVA to AVPI, i.e., mutating glutamine at site 8 into alanine, mutating arginine at site 9 into valine, mutating valine at site 10 into proline and mutating alanine at site 11 into isoleucine. Unlike the traditional protein improvement concept based on exogenous sequence fusion, the structural design of this mutein can obtain new property of original protein by use of endogenous mutation method, and maximally maintain the stability and bioactivity of protein without changing the length of the original protein sequence and spatial conformation.

Further, the cDNA sequence encoding the mutein is shown as SEQ ID NO: 1.

The second purpose of the invention is to provide a preparation method of the bi-targeted mutein MuR6S4TR of TRAIL, comprising the following steps:
A. Amplifying and cloning cDNA fragment;
B. Constructing and identifying expression vector;
C. Recombining the expression of the bi-targeted mutein of TRAIL;
D. Purifying the bi-targeted mutein of TRAIL;
E. Identifying the bi-targeted mutein of TRAIL;

Further, the sub-steps for constructing and identifying the expression vector in Step B comprise:
B₁. Excising corresponding fusion tagged sequence in the prokaryotic expression vector;
B₂. Cloning the optimized cDNA sequence encoding bi-targeted mutein of TRAIL on the prokaryotic expression vector to obtain high-efficient soluble non-fusion expression.

Further, the prokaryotic expression vector in Sub-step B₁ is pET 32a.

The induction temperature for expression of the recombinant protein in Step C is 18-30°C.

The sub-steps for purifying of the bi-targeted mutein of TRAIL in Step D comprise:
D₁. Firstly purifying with cation exchange resin to capture the target protein in supernatant after cell disruption;
D₂. Secondly purifying with high-density phenyl hydrophobe to further improve protein purity and remove endotoxin;
D₃. Finally refining and purifying with anion exchange resin to meet industrial amplification and further clinical application requirements.

The third purpose of the invention is to provide the bi-targeted mutein MuR6S4TR of TRAIL for use in antineoplastictherapy.

Additionally, the invention provides a method for manufacturing antineoplastic drugs using the bi-targeted mutein MuR6S4TR of TRAIL according to the invention.

For the effect mechanism of tumor cell apoptosis-inducing drugs, the bi-targeted mutein MuR6S4TR of TRAIL has cell-penetrating peptide-like mutability, and activity of the second mitochondrial-derived activator of caspase (Smac). The protein has clear synergism on double targets against the cellular exogenous apoptotic pathway for tumor cell apoptosis and the apoptosis inhibitor XIAP of multiple drug-resistant tumor cells, and has better effect on drug-resistant carcinoma.

The beneficial technical effects of the invention are as follows:
1. New protein structure. Mutations at fewer sites are used to minimally change the structure based on the existing mature protein precursors so as to preserve the spatial conformation and activity basis of the original wild-type protein, and greatly improve the structural stability and biological activity of the protein.
2. High protein expression level and soluble expression ratio. After the modification form of high-efficiency prokaryotic expression vector pET32a is used, the expression vector can be used to greatly obtain the expression level and soluble expression ratio higher than those of the wide-type protein of TRAIL within a wide temperature range from 18°C to 30°C.
3. Unlike the purification preparation process of the wild-type protein of TRAIL, the efficiency, recovery and product quality of the process are significantly improved. Since the purification method of specific affinity chromatography is not adopted, the corresponding reduction in purification cost can obviously amplify the potential to fully meet the future clinical requirements.
4. Extensive in vitro and in vivo biological activities. Compared with the wild-type protein of TRAIL, the antineoplastic activity of the bi-targeted mutein MuR6S4TR of TRAIL is significantly enhanced in almost all types of detected tumor cells, especially in TRAIL-resistant tumor cell strains, it can obviously reverse the resistance to the wild-type protein of TRAIL and has a stronger therapeutic effect. It is expected that MuR6S4TR can be used alone or in combination for treating drug-resistant colon (rectal) cancer, non-small cell lung cancer, breast cancer, liver cancer, pancreatic cancer and brain tumor.
5. Perfect action target selective combination. Typical tumor cell apoptosis receptor agonist TRAIL is selectively combined with mitochondria through 4 peptides at N-terminal of mitochondrial pathway apoptotic promoter Smac molecule after cell-penetrating peptide-like mutation. Extracellular receptor pathway and intracellular mitochondria pathway inducing tumor cell apoptosis are activated at the same time. The bi-targeted mutein MuR6S4TR of TRAIL enhances tumor cell apoptosis and strongly inhibits the activity of apoptotic antagonist XIAP against the key factor of TRAIL resistance mainly existing in multiple drug-resistant tumor cells, and has an ability of penetrating cytomembrane and entering cytoplasm. It can become multi-pathway anti-tumor mutein.

### Brief Description of the Drawings

In order to clearly describe the embodiments of the invention or the technical solution in the prior art, the embodiments or drawings used in technical description will be simply introduced as follows. Apparently, the drawings described below are only some embodiments of the invention. Those of ordinary skill in the art can obtain other drawings based on these drawings without creative work.

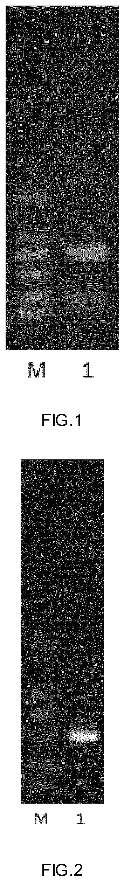

Fig. 1 is an electrophoretogram of PCR product on MuR6S4TR-1 fragment. Electrophoresis conditions: 1% Agarose, voltage of 150V, 25min. Lane 1: electrophoretic band of PCR product on MuR6S4TR-1; M: DL2000 (band molecular weight: 2000bp, 1000bp, 750bp, 500bp, 250bp and 100bp from the top down), loading amount: 5µl; loading amount of PCR product: 3µl.
Fig. 2 is an electrophoretogram of PCR product on MuR6S4TR fragment. Electrophoresis conditions: 1% Agarose, voltage of 150V, 25min. Lane 1: electrophoretic band of PCR product on MuR6S4TR; M: DL2000 (band molecular weight: 2000bp, 1000bp, 750bp, 500bp, 250bp and 100bp from the top down), loading amount: 5µl; loading amount of PCR product: 3µl.

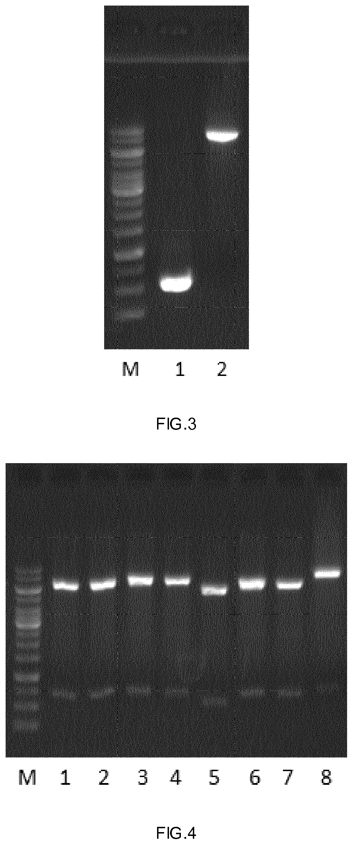

Fig. 3 is graphical electrophoresis result of gel extraction after enzyme digestion by Nde I and EcoR I. Electrophoresis conditions: 1% Agarose, voltage of 150V, 25min. Lane 1: electrophoretic band of gel extraction product after enzyme digestion by pET32a; electrophoretic band of gel extraction product after enzyme digestion by MuR6S4TR; M: GeneRuler 1kb DNA Ladder (band molecular weight: 10000bp, 8000bp, 6000bp, 5000bp, 4000bp, 3500bp, 3000bp, 2500bp, 2000bp, 1500bp, 1000bp, 750bp, 500bp and 250bp from the top down), loading amount: 5µl; loading amount of PCR product: 3µl.
Fig. 4 is a graphical result of enzyme digestion identification. Electrophoresis conditions: 1% Agarose, voltage of 150V, 30min. Lane 1-8: electrophoretogram after enzyme digestion by plasmid extracted from pET32a/ MuR6S4TR 1^{#}∼8^{#} strains; M: GeneRuler 1kb DNA Ladder (band molecular weight: 10000bp, 8000bp, 6000bp, 5000bp, 4000bp, 3500bp, 3000bp, 2500bp, 2000bp, 1500bp, 1000bp, 750bp, 500bp and 250bp from the top down). Loading amount of identified product: 10µl, loading amount of Marker: 5µl.

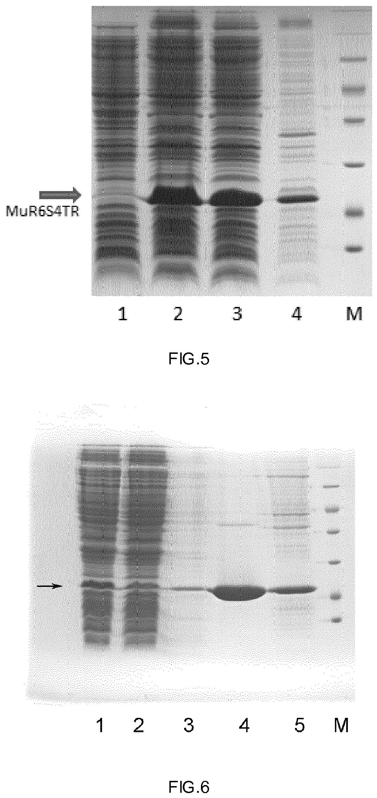

Fig. 5 is an electrophoretogram of pET32a/MuR6S4TR SDS-PAGE. Electrophoretic conditions: 15% gel, 200V, 35min. Lane 1: electrophoretic band before induction by pET32a/MuR6S4TR, Lane 2: electrophoretic band after induction by pET32a/MuR6S4TR, Lane 3: supernatant electrophoretic band after cell disruption by pET32a/MuR6S4TR, Lane 4: precipitation electrophoretic band after cell disruption by pET32a/MuR6S4TR, M: Unstained Protein Molecular Weight Marker (band molecular weight: 116.0KDa, 66.2KDa, 45.0KDa, 35.0KDa, 25.0KDa, 18.4KDa and 14.4KDa from the top down), loading amount of Marker: 5µl, loading amount of other samples: 20µl.
Fig. 6 is an SDS-PAGE electrophoretogram of cation exchange process. Electrophoretic conditions: 15% gel, 200V, 50min. Lane 1: feed liquid, Lane 2: penetrating liquid, Lane 3: step 1 eluent, Lane 4: step 2 eluent, Lane 5: NaOH eluent, M: Unstained Protein Molecular Weight Marker (band molecular weight: 116.0KDa, 66.2KDa, 45.0KDa, 35.0KDa, 25.0KDa, 18.4KDa and 14.4KDa from the top down). Loading amount of sample Marker: 5µl, loading amount of others: 20µl.

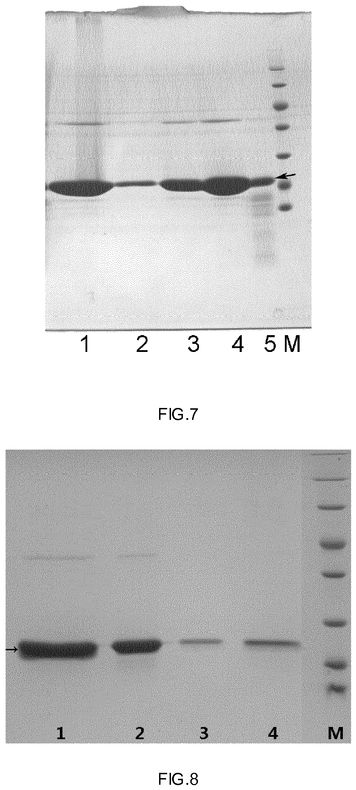

Fig. 7 is an SDS-PAGE electrophoretogram of high-density phenyl hydrophobic process. Electrophoretic conditions: 15% gel, 200V, 50min. Lane 1: feed liquid, Lane 2: penetrating liquid, Lane 3: step 1 eluent, Lane 4: step 2 eluent, Lane 5: NaOH eluent, M: Unstained Protein Molecular Weight Marker (band molecular weight: 116.0KDa, 66.2KDa, 45.0KDa, 35.0KDa, 25.0KDa, 18.4KDa and 14.4KDa from the top down). Loading amount of sample Marker: 5µl, loading amount of others: 20µl.
Fig. 8 is an SDS-PAGE electrophoretogram of anion exchange process. Electrophoretic conditions: 15% gel, 200V, 50min; Lane 1: anion exchange stock solution, Lane 2: anion exchange penetrating liquid, Lane 3: 2M NaCl eluent, Lane 4: 0.5M NaOH eluent, M: Unstained Protein Molecular Weight Marker (band molecular weight: 116.0KDa, 66.2KDa, 45.0KDa, 35.0KDa, 25.0KDa, 18.4KDa and 14.4KDa from the top down); loading amount of sample Marker: 5µl, loading amount of others: 20µl.

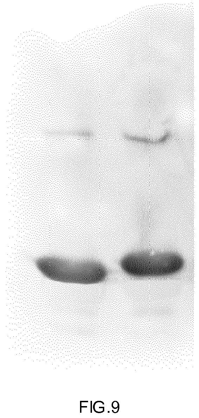

Fig. 9 is an identification diagram of MuR6S4TR by Western blot. Lane 1: Western blot result figure of supernatant after cell disruption by pET32a/MuR6S4TR; Lane 2: Western blot result figure of supernatant after cell disruption by pET32a/TRAIL.

### Detailed Description of the Preferred Embodiment

The technical solution in the embodiments of the invention will be described clearly and completely in combination with the figures of the embodiments as follows.

### Example 1

### Design of sequence and primer of bi-targeted mutein of TRAIL

The invention is to form 6 types of continuous arginine (RRRRRR) at N-terminal of mutein followed by the encoding sequence of 4 peptides (alanine, valine, proline and isoleucine (AVPI)) at N-terminal of Smac protein by selectively mutating the sequence of glutamine, arginine valine and alanine (QRVA) into the sequence of 4 peptides, i.e., alanine, valine, proline and isoleucine (AVPI) at N-terminal of Smac protein (4 mutation sites) from the amino acid sequence at site 8 to site 11 after RRRRRR (R6) at site 2 to site 7 in MuR6 sequence, based on cell-penetrating peptide-like mutant TRAIL-MuR6 of TRAIL (PCT/CN2015/073524: cell-penetrating peptide-like mutant MuR6 of TRAIL, preparation method and application) sequence. New mutein has cell-penetrating peptide-like mutability, and activity of the second mitochondrial-derived activator of caspase. The new mutein is named as bi-targeted mutein MuR6S4TR of TRAIL.

cDNA encoding mutein is shown as SEQ ID NO: 1, and the ammonic acid sequence of mutein is shown as SEQ ID NO: 2.

Primer synthesis is as follows:
Upstream primer:
   MuR6S4TR-Ndel-1: (see SEQ ID NO: 4) GGTCATATGCGTCGTCGTCGTCGTCGTGCTGTTCCGATTGCT
   MuR6S4TR-2: (see SEQ ID NO: 5) CGTCGTGCTGTTCCGATTGCTCACATCACTGGTAC
Downstream primer:
   TR-Eco-R: (see SEQ ID NO: 6) GTTGAATTCTTATTAACCAACAAGGAAAGCACCGAAGAAAG

### Example 2

Amplify MuR6S4TR gene fragment by PCR in two steps, then double-digest the segment and directly ligate with the expression vector pET32a subject to the same enzyme digestion, and pick and identify single colony of ligation product.

See Example 1 for primer design. The template of pET32a/MuR6TR is from patent PCT/CN2015/073524.

Specifically, the pET32a/MuR6TR cDNA sequence is shown in SEQ ID No: 3.

### Experimental steps

### I. Amplifying the target fragment of MuR6S4TR by PCR

1. Amplify the target fragment of MuR6S4TR-1 by MuR6S4TR-2/TR-Eco-R primer pair, and prepare the reaction system in accordance with Table 1 (reaction system: 50µl).
2. Uniformly mix the reaction system in vortex vibration manner, briefly centrifuge it and collect solution to the bottom of the tube.
3. See Table 2 for the PCR amplification reaction condition.
4. Carry out electrophoresis and take a picture.
5. Purify the sample of the target fragment MuR6S4TR-1 amplified by PCR in Omega PCR purification kit, elute it with 30µl ultrapure water, carry out electrophoresis and take a picture, and take it as a template for the second round of PCR.
6. Amplify the target fragment of MuR6S4TR by MuR6S4TR-Ndel-1/TR-Eco-R primer pair, and prepare the reaction system in accordance with Table 3 (reaction system: 50µl).
7. Uniformly mix the reaction system in vortex vibration manner, briefly centrifuge it and collect solution to the bottom of the tube.
8. See Table 4 for the PCR amplification reaction condition.
9. Carry out electrophoresis and take a picture.
10. Extract gel from the target fragment MuR6S4TR amplified by PCR in Omega PCR gel extraction kit, elute it with 50µl ultrapure water, carry out electrophoresis and take a picture for standby.

**Table 1 MuR6S4TR-1 PCR reaction system**

| Reagent | 50µl reaction system |
|---|---|
| Template: plasmid DNA | pET32a/MuR6S4TR (1µl) |
| 10×PCR Buffer for KOD-Plus-Neo | 5µl |
| dNTPs (2mM each) | 5µl |
| 25mM MgSO₄ | 3µl |
| KOD-Plus-Neo | 1µl |
| Primer pair MuR6S4TR-2/TR-Eco-R (10pmol/µl each) | 1µl each |
| RNase-Free Water | 33µl |

**Table 2 MuR6S4TR-1 PCR reaction system**

| Step | Temperature | Time | | |
|---|---|---|---|---|
| Predegeneration | 94°C | 2min | | |
| Degeneration | 94°C | 15s | 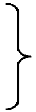 | 25 cycles |
| Annealing/extension | 68°C | 30s | | |
| Final extension | 68°C | 5min | | |

**Table 3 MuR6S4TR PCR reaction system**

| | 50µl reaction system |
|---|---|
| MuR6S4TR -1 PCR purification product | 1µl |
| 10×PCR Buffer for KOD-Plus-Neo | 5µl |
| dNTPs (2mM each) | 5µl |
| 25mM MgSO₄ | 3µl |
| KOD-Plus-Neo | 1µl |
| Primer pair MuR6S4TR-Ndel-1/TR-Eco-R (10pmol/µl each) | 1µl each |
| RNase-Free Water | 33µl |

**Table 4 MuR6S4TR PCR reaction system**

| Step | Temperature | Time | | |
|---|---|---|---|---|
| Predegeneration | 94°C | 2min | | |
| Degeneration | 94°C | 15s | 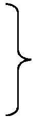 | 25 cycles |
| Annealing/extension | 68°C | 30s | | |
| Final extension | 68°C | 5min | | |

### II. Ligation of target fragment MuR6S4TR and pET32a plasmid after double enzyme digestion.

1. Double-digest the vector and target gene fragment with Ndel and EcoRI, and see Table 5 for enzyme digestion system (reaction system: 100µl).
2. Put EP tube into a multi-purpose thermostat at 30°C for 2h.
3. Extract gel by a gel extraction kit of OMEGA, and elute the vector and target fragment with 30µl ultrapure water respectively. Carry out electrophoresis and take a picture.
4. Ligate the gel extracted target fragment and vector, and see Table 6 for the ligation system.
5. Incubate the ligation system in a metal bath at 16°C overnight.
6. Add 100µl Top10 competent cells to 10µl ligation product in ice bath for 30min.
7. Carry out thermal shock in water batch at 42°C for 90s.
8. Place the solution on ice and incubate it for 2 min.
9. Add 500µl SOC culture medium, and oscillate and culture it at 37°C for 45min.
10. Centrifuge transformed competent cells, remove 400µl culture medium from clean bench, remaining about 100µl culture medium, and blow bacteria well, fully coat it on LB solid culture medium containing AMP and culture it at 37°C overnight.

**Table 5 Enzyme digestion reaction system of vector and target gene**

| Reagent | Volume | |
|---|---|---|
| DNA name | pET32a | Target gene fragment MuR6S4TR |
| DNA | 50µl | 45µl |
| Nde I | 5µl | 3µl |
| EcoR I | 5µl | 3µl |
| 10xH Buffer | 10µl | 10µl |
| dH₂O | 30µl | 39µl |
| Total | 100µl | 100µl |

**Table 6 Ligation reaction system of vector and target gene**

| Reagent | 10µl reaction system |
|---|---|
| Vector (pET32a) | 1µl |
| Target gene fragment MuR6S4TR | 4µl |
| Ligase (sol I) | 5µl |

### III. Picking of single colony and enzyme digestion identification

### (I) Picking of single colony

1. Prepare 40 sterilized test tubes and 100ml LB culture medium containing ampicillin.
2. Keep portions of culture medium in all test tubes, about 4ml for each tube.
3. Clamp sterile gun tip with fully burnt tweezers from a dish containing colonies, and select 8 colonies from the flat plate. Put the gun tip into the test tube filled with LB culture medium.
4. Bundle all test tubes and fully fix them on a shaker fixture. Vibrate them at 37°C and at 220rpm overnight.

### (II) Plasmid extraction

1. Separately take 1ml of bacteria solution into a centrifuge tube. Centrifuge the tube at 10000×g for 1min, and absorb supernatant as much as possible.
2. Add 250µl Solution I (add RNAase A in advance) to the centrifuge tube containing bacteria precipitate, and thoroughly suspend bacteria precipitate.
3. Add 250µl Solution II, gently and uniformly mix them for adequate bacteria pyrolysis, change the bacteria solution into a clear and sticky solution, and complete such step within 5min.
4. Add 350µl Solution III to the centrifuge tube, immediately reverse the tube and uniformly mix the solution, centrifuge it above 13000xg for 10min when white flocculent appears. A precipitate forms at the bottom of the centrifuge tube at the same time.
5. Uniformly add the supernatant obtained in Step 5 to two HiBind Miniprep adsorption columns placed in a collecting tube, do not extract the precipitate out, but centrifuge it at 10000×g for 1min, remove the waste liquid from the collecting tube and re-place the adsorption columns back to the collecting tube.
6. Add 500µl Buffer HB to the collecting tube, centrifuge it at 10000xg for 1min, remove the waste liquid from the collecting tube and put the adsorption columns back to the collecting tube.
7. Add 700µl Wash Buffer to the collecting tube, centrifuge it at 10000×g for 1min, remove the waste liquid from the collecting tube and put the adsorption columns back to the collecting tube.
8. Repeat Step 7.
9. Put the adsorption columns back to the collecting tube, centrifuge it above 13000xg for 2min, dry the adsorption columns and remove the waste liquid from the collecting tube.
10. Put all adsorption columns in a new 1.5ml EP tube, add 65µl Elution Buffer dropwise to the intermediate part of each adsorption film, place it at room temperature for several minutes, centrifuge it at 13000xg for 1min, and collect plasmid solution to 1.5ml EP tube.
11. Obtain 60µl plasmid DNA. Keep the plasmid at -20°C.

### (III) Enzyme digestion identification

1. Double-digest pET32a/MuR6S4TR plasmid with Xba I and EcoR I. See Table 7 for enzyme digestion reaction system.
2. Put EP tube into a multi-purpose thermostat at 37°C and incubate it for 2h.
3. Carry out electrophoresis identification after enzyme digestion.

**Table 7 Enzyme digestion reaction system of pET32a/MuR6S4TR plasmid**

| Reagent | Volume |
|---|---|
| DNA (extracted plasmid) | 5µl |
| Xba I | 0.5µl |
| EcoR I | 0.5µl |
| 10×M Buffer | 1µl |
| dH₂O | 3µl |
| Total | 10µl |

### (IV) Select correctly digested and successfully ligated strain, and store glycerol strain for sequencing.

### Experimental results

### I. Result of target fragment amplified by PCR

1. MuR6S4TR-1 target gene fragment was amplified by MuR6S4TR-2/TR-Eco-R primer pair. The molecular weight of the fragment was about 500bp. The target gene was obtained according to the above PCR reaction condition, as shown in Fig. 1.
2. MuR6S4TR target gene fragment was amplified by MuR6S4TR-Ndel-1/TR-Eco-R primer pair. The molecular weight of the fragment was about 500bp. The target gene was obtained according to the above PCR reaction condition, as shown in Fig. 2.

### II. Ligation and transformation results of target fragment and pET32a

(I) In theory, the fragment sizes of MuR6S4TR and pET32a which were double-digested with Ndel and EcoRI were about 500bp and about 5.4Kb respectively, as shown in Fig. 3. A single band was obtained after enzyme digestion and gel extraction.
(II) Ligation and transformation results of MuR6S4TR and pET32a
   1. The density of the dish was normal when colony appeared.
   2. Single colony was picked. The density was normal that bacteria appeared in all test tubes the next day.
   3. The plasmid was identified by enzyme digestion method, pET32a/MuR6S4TR plasmid could be identified by double enzyme digestion with Xbal and EcoRI. The vector fragment of about 5.4Kb and the target fragment of about 550bp should appear after enzyme digestion of successfully ligated plasmid. As shown in the figure, 8 samples of pET32a/MuR6S4TR 1^{#}∼8^{#} are positive clones.

### Example 3

### Expression test of plasmid pET32a-MuR6S4TR

Correctly sequenced plasmid obtained from Example 2 was used to transform the competent Escherichia coli BL21 (DE3), and one single colony was selected for expression test, and the expression effect was observed.

### Experimental steps

### I. Plasmid transformation and strain storage

1. Prepare 100ml LB culture medium and sterilize it at 121°C for 20min.
2. Add 1µl pET32a-MuR6S4TR plasmid to 100µl BL21 (DE3) competent cells in ice bath for 30min.
3. Carry out thermal shock in water batch at 42°C for 90s.
4. Place the solution on ice and incubate it for 3 min.
5. Fully coat 20µl transformed competent cells on LB solid culture medium containing Amp, and culture it at 37°C overnight.
6. Pick two single colonies from the flat plate and add them to 50ml LB (Amp⁺) and culture them at 37°C overnight after colonies appear on the flat plate the next day.
7. Store 20 vials of glycerin bacteria (final concentration of glycerin: 15%) at -20°C.

### II. Strain expression

1. Inoculate 10000µl pET32a-MuR6S4TR culture solution cultured overnight to 50ml LB (Amp⁺) culture medium. Vibrate the solution at 37°C and at 250rpm after inoculation, and reduce the temperature to 24°C culturing it for 3h. Add 0.1M IPTG at ratio of 1% for induced culture, sample 0.5ml centrifugate before induction and remove supernatant, add 50µl H₂O heavy suspension and then add 50µl 2×loading buffer to prepare induced electrophoresis sample.
2. Collect bacteria after induction overnight, detect A600 value, sample 150µl centrifugate and remove supernatant, add 50µl H₂O heavy suspension and then add 50µl 2×loading buffer to prepare induced electrophoresis sample, use Type 5430R centrifuge for the remaining bacteria solution, and centrifuge it at 12000rpm for 5min.
3. Take 50ml culture solution and centrifuge it to obtain bacteria, use 8ml 50mM Na₂HPO₄ solution for suspension and disrupt cells by ultrasonic wave. Cell disruption conditions: use φ6 probe, disrupt cells by 200W pulse for 2s and then stop for 2s, do circulation for totally 10min.
4. Take 1ml cell disruption solution at 12000rpm and centrifuge it for 10min, separate supernatant from precipitate, use 1ml H₂O for suspension of precipitate, separately take 20µl supernatant and precipitate suspension, and add 30µl H₂O and 50µl 2×loading buffer to prepare electrophoresis sample.
5. Place the prepared electrophoresis sample in boiling water bath for 10min, use Type 5430R centrifuge with Type A-45-30-11 rotor, centrifuge it at 12000rpm for 10min, and take 10µl supernatant for electrophoresis.

### Experimental results

Fig. 5 electrophoretogram shows that the expression of pET32a-MuR6S4TR of single colony is high.

| **Sample name** | **Total expression** | **Supernatant expression** | **Precipitate expression** |
|---|---|---|---|
| MuR6S4TR | High | 80% | 20% |

The experimental results showed that the expression of pET32a-MuR6S4TR target protein was high, and the supernatant contained about 80% target protein that was convenient for separation and purification after cell disruption.

### Example 4

### Purification preparation of MuR6S4TR protein

According to a large number of trials of MuR6S4TR process, we established a purification process of MuR6S4TR protein, and purified the MuR6S4TR protein by three-step method using SP-HP cation exchange, high-density phenyl hydrophobic and anion exchange breakthrough to obtain samples for both in vitro and in vivo activity analysis.

### Experimental steps

### I. Cell disruption and centrifugation

1. Take 30g MuR6S4TR expression bacteria, add Na₂HPO₄- NaH₂PO₄, glycerin, Tween 20, DTT and NaCl, make final concentration of above substances to be 20mM, 5%, 0.1%, 1mM, 600mM respectively, and additionally add H₂O to add the total volume to 80ml.
2. Carry out cell disruption for bacteria solution by ultrasonic wave. Cell disruption: use φ10 probe, disrupt cells by 500W pulse for 2s and then stop for 2s, disrupt cells for totally 15min.
3. Use Type 5430R centrifuge with Type F-35-6-30 rotor, centrifuge it at 7850rpm for 40min, take supernatant and use it as column loading sample after being filtered by 0.45µm filter membrane.

### II. Purification solution of protein and column preparation

1. Prepare the following solutions:
   (1) Cation exchange buffer solution A: 20mM Na₂HPO₄- NaH₂PO₄, 800mM NaCl, 5% glycerin, 0.1%Tween 20, 1mM DTT. Adjust pH to 7.0.
   (2) Cation exchange buffer solution B: 20mM Na₂HPO₄- NaH₂PO₄, 1M NaCl, 5% glycerin, 0.1%Tween 20, 1mM DTT. Adjust pH to 7.0.
   (3) Cation exchange eluent: 20mM Na₂HPO₄- NaH₂PO₄, 1.4M NaCl, 5% glycerin, 0.1%Tween 20, 1mM DTT. Adjust pH to 7.0.
   (4) 0.5M NaOH.
   (5) Phenyl hydrophobic buffer solution A: 20mM Na₂HPO₄- NaH₂PO₄, (NH₄)₂SO₄ with 30% saturation, 1.4M NaCl, 5% glycerin, 0.1%Tween 20, 1mM DTT. Adjust pH to 7.0.
   (6) Phenyl hydrophobic buffer solution B: 20mM Na₂HPO₄- NaH₂PO₄, (NH₄)₂SO₄ with 24% saturation, 1.4M NaCl, 5% glycerin, 0.1%Tween 20, 1mM DTT. Adjust pH to 7.0.
   (7) Cation exchange eluent: 20mM Na₂HPO₄- NaH₂PO₄, 700mM NaCl, 5% glycerin, 0.1%Tween 20, 1mM DTT. Adjust pH to 7.0.
   (8) Anion exchange buffer solution: 20mM Na₂HPO₄-NaH₂PO₄, 60mM NaCl, 0.3M glycine. Adjust pH to 7.0.
2. Use SP Sepharose Fast Flow gel chromatography column, flush ethanol left on the column with 5CV pure water, and then use 5CV corresponding equilibration buffer for equilibration.
3. Use MPC HCT XK16 Grad gel chromatography column, flush NaOH on the dilution column with 1CV pure water, use 5CV pre-equilibration buffer for equilibration and then use equilibration buffer for equilibration.
4. Use Sephadex G-25 medium gel chromatography column, flush ethanol left on the column with 5CV pure water, and then use 5CV anion exchange buffer solution for equilibration.
5. Use Q Sepharose Fast Flow gel chromatography column, flush ethanol left on the column with 5CV pure water, and then use 5CV anion exchange buffer solution for equilibration.

### III. Cation exchange purification

Carry out cation exchange purification in accordance with the following purification steps. Collect all penetration and elution compositions during purification period for electrophoretic analysis:
1. Equilibration: use cation exchange A buffer solution to equilibrate SP Sepharose Fast Flow chromatography column until UV becomes stable.
2. Sample preparation and loading: take supernatant which has been subject to cell disruption and centrifugation, and then load the sample.
3. Cleaning: use 2CV cation exchange buffer solution A to wash the column so as to remove the unbound residual protein.
4. Elution: use 3CV cation exchange buffer solution B to elute the target protein.
5. Cleaning of NaOH: use 2CV 0.5M NaOH solution to clean the column.
6. Reequilibration: use 5CV cation exchange buffer solution A to reequilibrate the column.

### IV. Phenyl hydrophobic purification

Carry out phenyl hydrophobic purification in accordance with the following purification steps. Collect all penetration and elution compositions during purification period for electrophoretic analysis:
1. Equilibration: use phenyl hydrophobic equilibration buffer to equilibrate MPC HCT XK16 Grad chromatography column until UV becomes stable.
2. Sample preparation and loading: add cation exchange eluent sample to (NH₄)₂SO₄ until its saturation is 30% and then load the sample.
3. Cleaning: use 2CV phenyl hydrophobic buffer solution B to wash the column so as to remove the unbound residual protein.
4. Elution: use 3CV phenyl hydrophobic eluent to wash the column and control pH.
5. Cleaning of NaOH: use 2CV 0.5M NaOH solution to elute the residual impurity and store the column.
6. Reequilibration: use 5CV phenyl hydrophobic buffer solution A to reequilibrate the column.

### V. Anion exchange purification

Carry out the third-step anion exchange purification in accordance with the following purification steps. Collect all penetration and elution compositions during purification period for electrophoretic analysis:
1. Equilibration: use anion exchange buffer solution to equilibrate Q Sepharose Fast Flow chromatography column until UV becomes stable.
2. Sample preparation and loading: take phenyl hydrophobic eluent sample, and load the sample after replacing the buffer solution with anion exchange buffer solution.
3. Cleaning of equilibrium liquid: use 1CV anion exchange buffer solution to clean the column to obtain the target protein of the unbound upper column.
4. Cleaning of NaCl: use 2CV 2M NaCl to wash the column so as to remove the protein bound on the column.
5. Cleaning of NaOH: use 2CV 0.5M NaOH solution to clean the column.
6. Reequilibration: use anion exchange buffer solution to reequilibrate the column.

### Experimental results

See Fig. 6, Fig. 7 and Fig. 8 for the electrophoresis results of the purification process sample of every step. 25ml target protein eluent for cation exchange purification was collected with concentration of 5.85.0mg/ml, and the tested target protein purity was 80.5%. Step 2: 23ml target protein eluent for high-density Phenyl hydrophobization was collected with concentration of 2.56mg/ml, and then tested target protein purity was 83.7%. Step 3: anion exchange penetrating liquid was 63ml with concentration of 0.9402mg/ml, and the target protein purity was 93.2.7%. The experimental steps of this example were repeated for several times to obtain a sufficient amount of protein for in vitro activity evaluation.

### Example 5

### Western Blot detection of MuR6S4TR protein

Since MuR6S4TR is obtained by mutation on site 10 at N-terminal of wild-type TRAIL, the antigenic determinant of TRAIL is still retained and could specifically bind to the polyclonal antibody of TRAIL. Therefore, the TRAIL polyclonal antibody can be used for detection and identification.

### Experimental steps

### I. Sample preparation

1. Dilute it with ultrapure water to be 1mg/ml according to its supplied concentration after unfreezing the MuR6S4TR protein purified in Example 4 from - 20°C. Add 50µl sample to 50µl 2×loading buffer to prepare electrophoresis sample. Separately take 10µl sample for electrophoresis, i.e., loading amount of 5ug.
2. Dissolve the reference substance TRAIL-20131204 freeze-dried product (own laboratory) with 1mlPBS, and add 50µl sample to 50µl 2×loading buffer to prepare electrophoresis sample. Separately take 10µl sample for electrophoresis, i.e., loading amount of 5ug.

### II. Detection process

Separate the sample through 15% SDS-PAGE electrophoresis and transfer membrane to PVDF membrane. Firstly, confine it at 4°C overnight, incubate it with primary antibody [rabbit anti-human TRAIL polyclonal antibody (1:500)] at room temperature for 2h, and then incubate it with secondary antibody [goat anti-rabbit IgG-HRP (1:5000)] at room temperature for 2h, and finally use enhanced chemiluminescence (ECL) for detection. The specific steps are as follows:
1. Separate protein by 15% SDS-PAGE electrophoresis; take out gel, cut off the gel edge and soak it in TBST buffer solution for 15min.
2. Use of PVDF membrane for membrane transfer (wet transfer): slightly soak the PVDF membrane with methanol before use for 15s, soak it in distilled water for 1-3 min and subsequently equilibrate it in the transmembrane buffer solution; in a transmembrane clip, lay foam-rubber cushion, filter papers (4-8 pieces), target gel, PVDF membrane, filter papers (4-8 pieces) and foam-rubber cushion in turns from cathode to anode, discharge bubbles and tighten the clamp in a transmembrane tank at voltage of 40V for 45min.
3. Confining membrane: confine the membrane in confining liquid (3%BSA) at 4°C overnight, take out it and shake it for 30min the next day to confine the nonspecific binding site.
4. Primary antibody incubation: dilute the primary antibody with confining liquid to the working concentration [rabbit anti-human TRAIL polyclonal antibody (1:500)], shake with the membrane and incubate them at room temperature for 2h.
5. Cleaning of membrane: clean membrane with TBST for three times, 10 min for each. Use more than 50ml cleaning solution for 10×10 cm membrane.
6. Secondary antibody incubation: dilute the secondary antibody marked with HRP with confining liquid to the working concentration [goat anti-rabbit IgG-HRP (1:5000)], shake with the membrane and incubate them at room temperature for 2h.
7. Cleaning of membrane: clean membrane with TBST for three times, 10 min for each. Use more than 50ml cleaning solution for 10×10 cm membrane.
8. Color development: (1) mix isovolumetric Solution A and Solution B, prepare sufficient mixed liquid for detection (0.125ml/cm²). Immediately use the mixed liquid for detection after preparation, and keep it stable at room temperature for 1h. (2) Drain off excess cleaning liquid cleaning the blotting membrane, but do not dry the membrane. Add the mixed liquid for detection on the membrane with protein on one face, drain off excess mixed liquid for detection, place it on Kodak gel imaging Image Station 4000R, expose it with X-ray for 1min firstly and adjust the exposure time in accordance with the image result. Record the image by a computer.
9. Result judgment: obvious chromatape should be shown in positive result. No chromatape is shown in negative result.

### Experimental results

As shown in Fig. 9, MuR6S4TR and TRAIL reference substances were positively reacted, and the negative control was negatively reacted.

### Example 7

### Analysis of bioactivity of proteins MuR6S4TR and TRAIL

CCK-8 detection kit was used to detect the in vitro anti-proliferative activity IC50 values of MuR6S4TR and wild-type TRAIL 2 protein samples on 11 tumor cell strains to evaluate in vitro biological activity.

### Materials and methods

The cell strains used for detection are from Shanghai Cell Bank of Chinese Academy of Sciences or American ATCC.

| | | Name of cell strain | Source of cell |
|---|---|---|---|
| 1 | Pancreatic cancer cell (3) | CFPAC-1 | Ordered from Shanghai Cell Bank of Chinese Academy of Sciences |
| 2 | | BxPC-3 | Ordered from Shanghai Cell Bank of Chinese Academy of Sciences |
| 3 | | PANC-1 | Ordered from Shanghai Cell Bank of Chinese Academy of Sciences |
| 4 | Lung cancer cell (2) | NCI-H460 | Ordered from Shanghai Cell Bank of Chinese Academy of Sciences |
| 5 | | A 549 | Ordered from Shanghai Cell Bank of Chinese Academy of Sciences |
| 6 | Colon (rectal) cancer (3) | SW620 | Ordered from Shanghai Cell Bank of Chinese Academy of Sciences |
| 7 | | HT-29 | Ordered from Shanghai Cell Bank of Chinese Academy of Sciences |
| 8 | | HCT 116 | Ordered from Shanghai Cell Bank of Chinese Academy of Sciences |
| 9 | Breast cancer cell (3) | MDA-MB-231 | Ordered from American ATCC |
| 10 | | MCF-7 | Ordered from American ATCC |
| 11 | | T47D | Ordered from American ATCC |

### Reagents and consumables

Cell Counting Kit-8 (Cat# CK04-13, Dojindo)
96-well culture plate (Cat# 3599, Corning Costar)
Fetal calf serum (Cat#10099-141, GIBCO)
Culture medium (purchased from GIBCO)
Desk microplate reader SpectraMax M5 Microplate Reader (Molecular Devices)
Two protein samples: Prepared by Example 4 or lab-supplied.

### Experimental steps

### 1. Reagent preparation

### Preparation of culture medium

| | | Name of cell strain | Culture medium and culture condition | Inoculation density |
|---|---|---|---|---|
| 1 | Pancreatic cancer cell (3) | CFPAC-1 | IMDM+10%FBS; CO₂, 5%; 37.0°C | 7 x10³/well |
| 2 | | BxPC-3 | RPMI-1640+10%FBS+1 mM sodium pyruvate; CO₂, 5%; 37.0°C | 4x10³/well |
| 3 | | PANC-1 | DMEM + 10%FBS; CO₂, 5%; 37.0°C | 5 x10³/well |
| 4 | Lung cancer cell (2) | NCI-H460 | RPMI-1640+10% FBS; CO₂, 5%; 37.0°C | 8 x10³/well |
| 5 | | A 549 | DMEM + 10%FBS; CO₂, 5%; 37.0°C | 5 x 10³/well |
| 6 | Colon (rectal) cancer cell (3) | SW620 | Leibovitz's L-15 + 10%FBS; without CO₂, 37.0°C | 8 x10³/well |
| 7 | | HT-29 | DMEM + 10% FBS; CO₂, 5%; 37.0°C | 5 x10³/well |
| 8 | | HCT 116 | DMEM + 10% FBS; CO₂, 5%; 37.0°C | 4 x10³/well |
| 9 | Breast cancer cell (3) | MDA-MB-231 | RPMI-1640+10% FBS ; CO₂, 5%; 37.0°C | 8 x10³/well |
| 10 | | MCF-7 | RPMI-1640+10% FBS; CO₂, 5%; 37.0°C | 8 x10³/well |
| 11 | | T47D | RPMI-1640+10% FBS+ 0.2 Units/ml bovine insulin; CO₂, 5%; 37.0°C | 10 x10³/well |

### Preparation of protein sample

Dilute 2 protein samples with sterile PBS buffer solution to obtain final concentration of 5 mg/ml, and filter and sterilize the samples.

### 2. IC50 experiment

a) Collect and count logarithmic growth phase cells, resuspend cells with complete culture medium, adjust the cell concentration to the appropriate concentration (as determined by the cell density optimization test results), inoculate 96-well plate and add 100 µl cell suspension to each well. Incubate cells (except SW620 cells, with no need for 5% CO₂) in 5% CO₂ incubator at 37°C and at 100% relative humidity for 24 h.
b) Dilute the protein sample to be tested with sterile PBS buffer solution to 5mg/m, carry out gradient dilution for 8 times and add cells based on 25µl/well. Dilute the final concentration of the compound from 1mg/ml to 0, 3 times gradually, totally 10 concentration points; correspondingly adjust the final action concentration of protein sample according to preliminary experimental result.
c) Incubate cells (except SW620 cells, with no need for 5% CO₂) in 5% CO₂ incubator at 37°C and at 100% relative humidity for 48 h.
d) Absorb the culture medium and add complete culture medium containing 10% CCK-8 and incubate it in a incubator 37°C for 2 to 4 hours.
e) Assay the absorbance at the wavelength of 450 nm on SpectraMax M5 Microplate Reader after slight vibration, and take the absorbance at 650 nm as a reference for calculation of inhibition rate.

### 3. Data processing

Calculate the inhibition rate of the drug on the growth of the tumor cells according to the following formula: percentage of growth inhibition rate of tumor cells= [(Ac-As) / (Ac-Ab)] x 100%
As: OA/RLU (cells+CCK-8+compound to be tested) of sample
Ac: OA/RLU (cells+CCK-8) for negative control
Ab: OA/RLU (culture medium+CCK-8) for positive control

Use the software Graphpad Prism 5 and the calculation formula log (inhibitor) vs. normalized response-Variable slope to fit the IC50 curve and calculate the IC50 value.

### Experimental results

In this study, the effects of two protein samples (MuR6S4TR and wild-type TRAIL) on the in vitro anti-cell proliferation activities of three pancreatic cancer cell strains (CFPAC-1, BxPC-3 and PANC-1), two lung cancer cell strains (NCI-H460 and A 549), 3 colon (rectal) cancer cell strains (SW620, HT-29, HCT 116) and 3 breast cancer cell strains (MDA-MB-231, MCF-7, T47D) were tested.

See the following table for the experimental results.

| | Cell type | Cell strains | MuR6S4TR (ug/ml) | TRAIL (ug/ml) |
|---|---|---|---|---|
| 1 | Pancreatic cancer (3) | BxPC-3 | 0.0616 | 0.2477 |
| 2 | | CFPAC-1 | 0.0231 | >100 |
| 3 | | PANC-1 | 0.0347 | >100 |
| 4 | Lung cancer (2) | A549 | 0.0777 | >100 |
| 5 | | NCl-H460 | 0.1153 | >100 |
| 6 | Colon cancer (3) | HCT116 | 0.0042 | 0.0030 |
| 7 | | HT-29 | 8.9675 | >100 |
| 8 | | SW620 | 4.3476 | >100 |
| 9 | Breast cancer (3) | MCF-7 | 0.0054 | >100 |
| 10 | | MDA-MB-231 | 0.0019 | 0.0091 |
| 11 | | T47D | 99.031 | >100 |

### Experimental conclusion

Compared with the TRAIL wild-type protein, the antineoplastic activity of the bi-targeted mutein MuR6S4TR of TRAIL is significantly enhanced in almost all types of detected tumor cells, especially in wild-type protein-resistant tumor cell strains of TRAIL, it can obviously reverse the resistance of these cells to the wild-type protein of TRAIL and has a stronger therapeutic effect.

## Claims

1. A bi-targeted mutein MuR6S4TR of TRAIL, wherein the amino acid sequence of the mutein is shown as SEQ ID NO: 2, wherein the mutein enables the site 2 to site 11 at N-terminal of mutein to become bi-targeted muteins containing cell-penetrating peptide sequence RRRRRR and binding sequence AVPI of apoptosis inhibitor XIAP by selectively mutating glutamine at site 8 into alanine in the amino acid sequence at site 8 to site 11 of TRAIL-MuR6, mutating arginine at site 9 into valine, mutating valine at site 10 into proline and mutating alanine at site 11 into isoleucine, i.e., mutating QRVA at site 8 to site 11 into AVPI sequence.

2. The bi-targeted mutein MuR6S4TR of TRAIL according to claim 1, wherein the cDNA sequence encoding the mutein is shown as SEQ ID NO: 1.

3. A preparation method of the bi-targeted mutein MuR6S4TR of TRAIL according to claim 1, comprising the following steps:
A. amplifying and cloning cDNA fragment;
B. constructing and identifying expression vector;
C. recombining the expression of the bi-targeted mutein of TRAIL;
D. purifying the bi-targeted mutein of TRAIL;
E. identifying the bi-targeted mutein of TRAIL;

4. The preparation method of the bi-targeted mutein MuR6S4TR of TRAIL according to claim 3, wherein the sub-steps for constructing and identifying the expression vector in Step B comprise:
B₁. excising fusion tagged sequence in the prokaryotic expression vector;
B₂. cloning the optimized cDNA sequence encoding bi-targeted mutein of TRAIL on the prokaryotic expression vector to obtain high-efficient soluble non-fusion expression.

5. The preparation method of the bi-targeted mutein MuR6S4TR of TRAIL according to claim 4, wherein the prokaryotic expression vector is pET 32a in Sub-step B₁.

6. The preparation method of the bi-targeted mutein MuR6S4TR of TRAIL according to claim 3, wherein the induction temperature for expression of the recombinant protein in Step C is 18-30°C.

7. The preparation method of the bi-targeted mutein MuR6S4TR of TRAIL according to claim 3, wherein the sub-steps for purifying of TRAIL protein in Step D comprise:
D₁. Firstly, purifying with cation exchange resin to capture the target protein in supernatant after cell disruption;
D₂. Secondly, purifying with high-density phenyl hydrophobe to further improve protein purity and remove endotoxin;
D₃. Finally, refining and purifying with anion exchange resin to meet industrial amplification and further clinical application requirements.

8. The bi-targeted mutein MuR6S4TR of TRAIL according any of claims 1 or 2 for use in antineoplastic therapy.

9. A method for manufacturing antineoplastic drugs using the bi-targeted mutein MuR6S4TR of TRAIL according any of claims 1 or 2.

## Patentansprüche

1. Doppelzielgerichtetes Mutein MuR6S4TR von TRAIL, wobei die Aminosäuresequenz des Muteins unter SEQ ID NO: 2 dargestellt ist, wobei das Mutein Stelle 2 bis Stelle 11 am N-Terminus des Muteins ermöglicht, zu doppelzielgerichteten Muteinen mit der zellpenetrierenden Peptidsequenz RRRRRR und der Bindungssequenz AVPI des Apoptosehemmers XIAP zu werden, indem selektiv Glutaminn an Stelle 8 zu Alanin in der Aminosäuresequenz an Stelle 8 bis Stelle 11 von TRAIL-MuR6 mutiert wird, Arginin an Stelle 9 zu Valin mutiert wird, Valine an Stelle 10 zu Prolin mutiert wird und Alanin an Stelle 11 zu Isoleucin mutiert wird, d. h. QRVA an Stelle 8 bis Stelle 11 zur AVPI-Sequenz mutiert wird.

2. Doppelzielgerichtetes Mutein MuR6S4TR von TRAIL nach Anspruch 1, wobei die das Mutein codierende cDNA-Sequenz unter SEQ ID NO: 1 dargestellt ist.

3. Herstellungsverfahren für das doppelzielgerichtete Mutein MuR6S4TR von TRAIL nach Anspruch 1, umfassend die folgenden Schritte:
A. Amplifizieren und Klonieren eines cDNA-Fragments;
B. Konstruieren und Identifizieren eines Expressionsvektors;
C. Rekombinieren der Expression des doppelzielgerichteten Muteins von TRAIL;
D. Aufreinigen des doppelzielgerichteten Muteins von TRAIL;
E. Identifizieren des doppelzielgerichteten Muteins von TRAIL;

4. Herstellungsverfahren für das doppelzielgerichtete Mutein MuR6S4TR von TRAIL nach Anspruch 3, wobei die Teilschritte zum Konstruieren und Identifizieren des Expressionsvektors in Schritt B Folgendes umfassen:
B₁.Ausschneiden der Sequenz mit Fusions-Tag im prokaryontischen Expressionsvektor;
B₂. Klonieren der optimierten doppelzielgerichtetes Mutein von TRAIL codierenden cDNA-Sequenz auf dem prokaryontischen Expressionsvektor unter Erhalt hocheffizienter löslicher Nicht-Fusion-Expression.

5. Herstellungsverfahren für das doppelzielgerichtete Mutein MuR6S4TR von TRAIL nach Anspruch 4, wobei es sich bei dem prokaryotischen Expressionsvektor in Teilschritt B₁. um pET 32a handelt.

6. Herstellungsverfahren für das doppelzielgerichtete Mutein MuR6S4TR von TRAIL nach Anspruch 3, wobei die Induktionstemperatur für Expression des rekombinanten Proteins in Schritt C bei 18-30°C liegt.

7. Herstellungsverfahren für das doppelzielgerichtete Mutein MuR6S4TR von TRAIL nach Anspruch 3, wobei die Teilschritte zur Aufreinigung von TRAIL-Protein in Schritt D Folgendes umfassen:
D₁. erstens Aufreinigen mit Kationenaustauschharz zum Einfangen des Zielproteins im Überstand nach Zellaufschluss;
D₂. zweitens Aufreinigen mit Phenylhydrophob hoher Dichte zur weiteren Verbesserung der Proteinreinheit und Abtrennung von Endotoxin;
D₃. schließlich Verfeinern und Aufreinigen mit Anionenaustauschharz, um die Erfordernisse von großtechnischer Amplifikation und weiteren klinischen Anwendungen zu erfüllen.

8. Doppelzielgerichtetes Mutein MuR6S4TR von TRAIL nach einem der Ansprüche 1 oder 2 zur Verwendung in der antineoplastischen Therapie.

9. Verfahren zur Erzeugung antineoplastischer Arzneistoffe unter Verwendung des doppelzielgerichteten Muteins MuR6S4TR von TRAIL nach einem der Ansprüche 1 oder 2.

## Revendications

1. Mutéine biciblée MuR6S4TR de TRAIL, la séquence d'acides aminés de la mutéine étant décrite par SEQ ID NO: 2, la mutéine permettant au site 2 au site 11 à l'extrémité N-terminale de la mutéine de devenir des mutéines biciblées contenant la séquence peptidique de pénétration cellulaire RRRRRR et la séquence de liaison AVPI d'inhibiteur d'apoptose XIAP par mutation sélective de la glutamine au site 8 en alanine dans la séquence d'acides aminés au site 8 au site 11 de TRAIL-MuR6, la mutation de l'arginine au site 9 en valine, la mutation de valine au site 10 en proline et la mutation d'alanine au site 11 en isoleucine, c'est-à-dire, la mutation de QRVA au site 8 au site 11 en séquence AVPI.

2. Mutéine biciblée MuR6S4TR de TRAIL selon la revendication 1, la séquence d'ADNc codant pour la mutéine étant décrite par SEQ ID NO: 1.

3. Procédé de préparation de la mutéine biciblée MuR6S4TR de TRAIL selon la revendication 1, comprenant les étapes suivantes :
A. amplification et clonage d'un fragment d'ADNc ;
B. construction et identification d'un vecteur d'expression ;
C. recombinaison de l'expression de la mutéine biciblée de TRAIL ;
D. purification de la mutéine biciblée de TRAIL ;
E. identification de la mutéine biciblée de TRAIL.

4. Procédé de préparation de la mutéine biciblée MuR6S4TR de TRAIL selon la revendication 3, dans lequel les sous-étapes de construction et d'identification du vecteur d'expression dans l'étape B comprennent :
B₁. l'excision de séquence étiquetée de fusion dans le vecteur d'expression procaryote ;
B₂. le clonage de la séquence d'ADNc optimisée codant pour la mutéine biciblée de TRAIL sur le vecteur d'expression procaryote pour obtenir une expression non-fusion soluble très efficace.

5. Procédé de préparation de la mutéine biciblée MuR6S4TR de TRAIL selon la revendication 4, dans lequel le vecteur d'expression procaryote est pET 32a dans la sous-étape B₁.

6. Procédé de préparation de la mutéine biciblée MuR6S4TR de TRAIL selon la revendication 3, dans lequel la température d'induction pour l'expression de la protéine recombinante dans l'étape C est de 18 à 30 °C.

7. Procédé de préparation de la mutéine biciblée MuR6S4TR de TRAIL selon la revendication 3, dans lequel les sous-étapes de purification de protéine TRAIL dans l'étape D comprennent :
D₁. premièrement, la purification avec une résine d'échange de cations pour capturer la protéine cible dans le surnageant après rupture des cellules ;
D₂. deuxièmement, la purification avec phényle hydrophobe à haute densité pour améliorer plus avant la pureté de la protéine et éliminer les endotoxines ;
D₃. finalement, le raffinage et la purification avec une résine d'échange d'anions pour satisfaire aux exigences d'amplification industrielle et aux autres exigences d'application clinique.

8. Mutéine biciblée MuR6S4TR de TRAIL selon l'une quelconque des revendications 1 ou 2 pour utilisation en thérapie antinéoplasique.

9. Procédé de fabrication de médicaments antinéoplasiques au moyen de la mutéine biciblée MuR6S4TR de TRAIL selon l'une quelconque des revendications 1 ou 2.


### SEQUENCE LISTING

<110> CHENGDU HUACHUANG Biotechnology CO.,LTD
<120> Bi-targeted mutain MuR6S4TR of TRAIL and preparation method and
   application thereof
<160> 6
<170> PatentIn version 3.3
<210> 1
   <211> 510
   <212> DNA
   <213> Artificial Sequence
<400> 1
   atgcgtcgtc gtcgtcgtcg tgctgttccg attgctcaca tcactggtac tcgtggtcgt      60
   tctaacactc tttcttctcc gaactctaaa aacgaaaaag ctcttggtcg taaaatcaac     120
   tcttgggaat cttctcgttc tggtcactct ttcctttcta accttcacct tcgtaacggt     180
   gaacttgtta tccacgaaaa aggtttctac tacatctact ctcagactta cttccgtttc     240
   caggaagaaa tcaaagaaaa cactaaaaac gataaacaga tggttcagta catctacaaa     300
   tacacctctt acccggaccc gatccttctt atgaaatctg ctcgtaactc ttgctggtct     360
   aaagatgctg aatacggtct ttactctatc taccagggtg gtatcttcga acttaaagaa     420
   aacgatcgta tcttcgtttc tgttactaac gaacacctta tcgatatgga tcacgaggct     480
   tctttcttcg gtgctttcct tgttggttaa                                      510
<210> 2
   <211> 169
   <212> PRT
   <213> Artificial Sequence
<400> 2
   Met Arg Arg Arg Arg Arg Arg Ala Val Pro Ile Ala His Ile Thr Gly
   1               5                   10                  15
   Thr Arg Gly Arg Ser Asn Thr Leu Ser Ser Pro Asn Ser Lys Asn Glu
               20                  25                  30
   Lys Ala Leu Gly Arg Lys Ile Asn Ser Trp Glu Ser Ser Arg Ser Gly
           35                  40                  45
   His Ser Phe Leu Ser Asn Leu His Leu Arg Asn Gly Glu Leu Val Ile
       50                  55                  60
   His Glu Lys Gly Phe Tyr Tyr Ile Tyr Ser Gln Thr Tyr Phe Arg Phe
   65                  70                  75                  80
   Gln Glu Glu Ile Lys Glu Asn Thr Lys Asn Asp Lys Gln Met Val Gln
                   85                  90                  95
   Tyr Ile Tyr Lys Tyr Thr Ser Tyr Pro Asp Pro Ile Leu Leu Met Lys
               100                 105                 110
   Ser Ala Arg Asn Ser Cys Trp Ser Lys Asp Ala Glu Tyr Gly Leu Tyr
           115                 120                 125
   Ser Ile Tyr Gln Gly Gly Ile Phe Glu Leu Lys Glu Asn Asp Arg Ile
       130                 135                 140
   Phe Val Ser Val Thr Asn Glu His Leu Ile Asp Met Asp His Glu Ala
   145                 150                 155                 160
   Ser Phe Phe Gly Ala Phe Leu Val Gly
                   165
<210> 3
   <211> 513
   <212> DNA
   <213> Artificial Sequence
<400> 3
   atgcgtcgtc gtcgtcgtcg tcagcgtgtg gctgctcaca tcactggtac tcgtggtcgt      60
   tctaacactc tttcttctcc gaactctaaa aacgaaaaag ctcttggtcg taaaatcaac     120
   tcttgggaat cttctcgttc tggtcactct ttcctttcta accttcacct tcgtaacggt     180
   gaacttgtta tccacgaaaa aggtttctac tacatctact ctcagactta cttccgtttc     240
   caggaagaaa tcaaagaaaa cactaaaaac gataaacaga tggttcagta catctacaaa     300
   tacacctctt acccggaccc gatccttctt atgaaatctg ctcgtaactc ttgctggtct     360
   aaagatgctg aatacggtct ttactctatc taccagggtg gtatcttcga acttaaagaa     420
   aacgatcgta tcttcgtttc tgttactaac gaacacctta tcgatatgga tcacgaggct     480
   tctttcttcg gtgctttcct tgttggttaa taa                                  513
<210> 4
   <211> 42
   <212> DNA
   <213> Artificial Sequence
<400> 4
   ggtcatatgc gtcgtcgtcg tcgtcgtgct gttccgattg ct 42
<210> 5
   <211> 35
   <212> DNA
   <213> Artificial Sequence
<400> 5
   cgtcgtgctg ttccgattgc tcacatcact ggtac 35
<210> 6
   <211> 41
   <212> DNA
   <213> Artificial Sequence
<400> 6
   gttgaattct tattaaccaa caaggaaagc accgaagaaa g 41